# FASCICULE DE BREVET EUROPEEN

(11) **EP 1 264 470 B1**
(45) Date de publication et mention de la délivrance du brevet: **13.05.2009**
(21) Numéro de dépôt: 01913961.7
(22) Date de dépôt: 08.03.2001
(51) Int. Cl.: H04M 11/04, A61B 5/00

(54) **SYSTEME DE TELE-ASSISTANCE MEDICALE A DOMICILE**
SYSTEM ZUR HÄUSLICHEN MEDIZINISCHEN FERNHILFE
HOME-BASED REMOTE MEDICAL ASSISTANCE

(30) Priorité: 16.03.2000 FR 0003367
(43) Date de publication de la demande: 11.12.2002
(73) Titulaire: FRANCE TELECOM, 75015 Paris (FR)
(72) Inventeur: PROVOST, Hervé, F-38170 Seyssinet-Pariset (FR); BERENGUER, Marc, F-38420 Revel (FR)
(74) Mandataire: Lemoyne, Didier
(86) Numéro de dépôt international: PCT/FR2001/000692
(87) Numéro de publication internationale: WO 2001/069906

(56) Documents cités:
- WO-A-98/40009
- US-A- 5 544 649

## Description

La présente invention concerne un système de télé-assistance médicale à domicile d'un patient.

L'invention trouve une application particulièrement avantageuse dans tous les domaines de l'assistance à domicile, que ce soit le maintien à domicile, les soins infirmiers à domicile ou l'hospitalisation à domicile, et, plus généralement, tous moyens qui permettent à des patients résidant dans leur cadre de vie habituel de bénéficier d'une surveillance et d'une assistance médicales.

Ceci s'applique notamment à des patients souffrant de pathologies nécessitant un suivi médical régulier, avec mise en oeuvre d'appareils d'assistance médicale dédiés, en dehors d'un séjour hospitalier. Cette prise en charge passe, par exemple, par l'utilisation de concentrateurs d'oxygène en oxygénothérapie, de pompes à nutrition, de pompes à chimiothérapie, de capteurs susceptibles de générer des alertes ou des alarmes sur l'état du patient ou sur les appareils placés à ses côtés.

On connaît des systèmes permettant de contrôler l'observance d'un traitement, en oxygénothérapie par exemple, qui rendent compte à distance de données mesurées sur les appareils médicaux. D'autres systèmes offrent la possibilité de téléprogrammer le matériel. D'autres encore peuvent envoyer des alertes ou des alarmes sur l'état du patient et/ou sur l'état des appareils disposés: à proximité, ce sont des systèmes de téléalarme se déclenchant sur l'action du patient lui-même ou sur un événement généré par un capteur.

Toutefois, ces systèmes connus ne donnent que peu confiance aux soignants sur ce qui se passe effectivement au domicile du patient.

Dans le cas des systèmes rendant compte à distance de données mesurées, le soignant ne sait qu'après coup que le traitement a été mal observé et, à distance, n'a que peu de moyens pour guider/éduquer le patient vers une meilleure observance.

Dans le cas de systèmes permettant de téléprogrammer les appareils, seules certaines données sont retournées faisant office d'accusé de réception de la téléprogrammation. Eventuellement, s'il existe un afficheur sur l'appareil distant, le patient peut corroborer par liaison téléphonique un changement d'état de l'appareil. Mais, même avec ce témoignage, le soignant ne peut pas s'assurer du bon fonctionnement global de l'installation (inspection des tubulures notamment).

Enfin, les systèmes d'alerte ou d'alarme classiques n'autorisent pas d'action à distance sur la cause de l'alerte ou de l'alarme, et, sans plus d'information, nécessitent le déglacement du soignant qui peut avoir,un doute de la validité de l'alerte ou de l'alarme ainsi que sur l'état du patient.

Aussi, le problème technique à résoudre par l'objet de la présente invention est de proposer un système de télé-assistance médicale à domicile d'un patient, qui permettrait notamment de garantir une conformité entre le fonctionnement attendu des appareils à proximité du patient et leur fonctionnement réel, de réaliser une commande à distance des appareils afin de pouvoir modifier leurs paramètres de fonctionnement pour les adapter aux besoins du patient, et de donner un maximum de confort et de confiance au soignant sur ce qu'il fait à distance.

La solution au problème technique consiste, selon la présente invention, en ce que ledit système comprend :
- une station-patient comprenant, au moins, une caméra vidéo-patient télécommandable utilisée pour fournir des images d'appareils d'assistance médicale, et un ordinateur-patient équipé, d'une part; de moyens d'acquisition de données en provenance d'appareils d'assistance médicale et de moyens de transmission d'ordres de commande auxdits appareils, et, d'autre part, de moyens de connexion à un réseau de télécommunication,
- une station-soignant comprenant, au moins, un écran de visualisation apte à recevoir des images en provenance de ladite caméra vidéo-patient, et un ordinateur-soignant muni de moyens de connexion audit réseau de télécommunication afin d'établir une communication avec la station-patient, de moyens de télépilotage de la station-patient aptes à télécommander la caméra vidéo-patient et à envoyer à l'ordinateur-patient lesdits ordres de commande, et de moyens de contrôle et de rétrocontrôle après télépilotage des effets desdits ordres de commandes émis, par visualisation sur ledit écran de visualisation des images desdits appareils fournies par la caméra vidéo-patient et par transfert dans l'ordinateur-soignant des données transmises depuis les moyens d'acquisition de l'ordinateur-patient à travers le réseau de télécommunication.

Ainsi, le système de télé-assistance selon l'invention permet de contrôler à distance le fonctionnement des appareils chez le patient, d'une part, de manière visuelle à l'aide de la caméra vidéo-patient pour s'assurer du bon état de l'installation du patient dans son ensemble, tubulures des appareils notamment, ou pour lire des indications fournies par des afficheurs, et, d'autre part, par transfert de données depuis les moyens d'acquisition de l'ordinateur-patient jusqu'à l'ordinateur-soignant. De plus, il est possible pour le soignant de modifier le fonctionnement des appareils en envoyant des ordres de commande via les moyens de télépilotage de l'ordinateur-soignant et les moyens de transmission de l'ordinateur-patient. Enfin, par le rétrocontrôle que permet le système conforme à l'invention, le soignant peut vérifier, visuellement ou par transfert de données, l'effet des actions qu'il a effectuées sur les appareils.

De manière pour le patient de voir le soignant au cours de la communication entre les deux stations, l'invention prévoit que la station-patient comprend également un écran de visualisation apte à recevoir des images en provenance d'une caméra vidéo-soignant située à la station-soignant. De même, afin de permettre l'établissement d'un échange entre patient et soignant, la station-patient et la station-soignant comprennent des moyens de communication phonique. Ces dispositions ont l'avantage de placer à distance les deux personnes concernées dans des conditions de dialogue particulièrement importantes pour le moral du patient

Deux modes de mise en communication de la station-patient avec la station-soignant sont envisagés. Selon un premier mode, une communication avec la station-soignant est établie volontairement par le patient, ou, selon un deuxième mode, une communication avec la station-soignant est déclenchée par une alarme sur un écart de fonctionnement ou panne d'un desdits appareils d'assistance médicale.

Une autre caractéristique avantageuse du système de télé-assistance, objet de l'invention, concerne la situation de fonctionnement en mode dégradé dans laquelle la station-patient se trouverait dans l'impossibilité d'entrer en communication avec la station-soignant, par exemple en dehors des heures d'ouverture d'un service d'hospitalisation à domicile. Dans ce cas, il est prévu par l'invention que ledit système comprend en outre un centre-relais apte à entrer en communication phonique avec un générateur vocal au domicile du patient.

La description qui va suivre en regard des dessins annexés, donnés à titre d'exemple non limitatifs, fera bien comprendre en quoi consiste l'invention et comment elle peut être réalisée.
La figure 1 est un schéma d'une station-patient et d'une station-soignant d'un système de télé-assistance médicale à domicile conforme à l'invention.
La figure 2 est un schéma de fonctionnement du système de la figure 1 lors d'une communication entre les stations.
La figure 3 est un schéma de fonctionnement du système de la figure 1 lors d'une alarme déclenchée au domicile du patient.
La figure 4 est un schéma de fonctionnement du système de la figure 1 en mode dégradé.

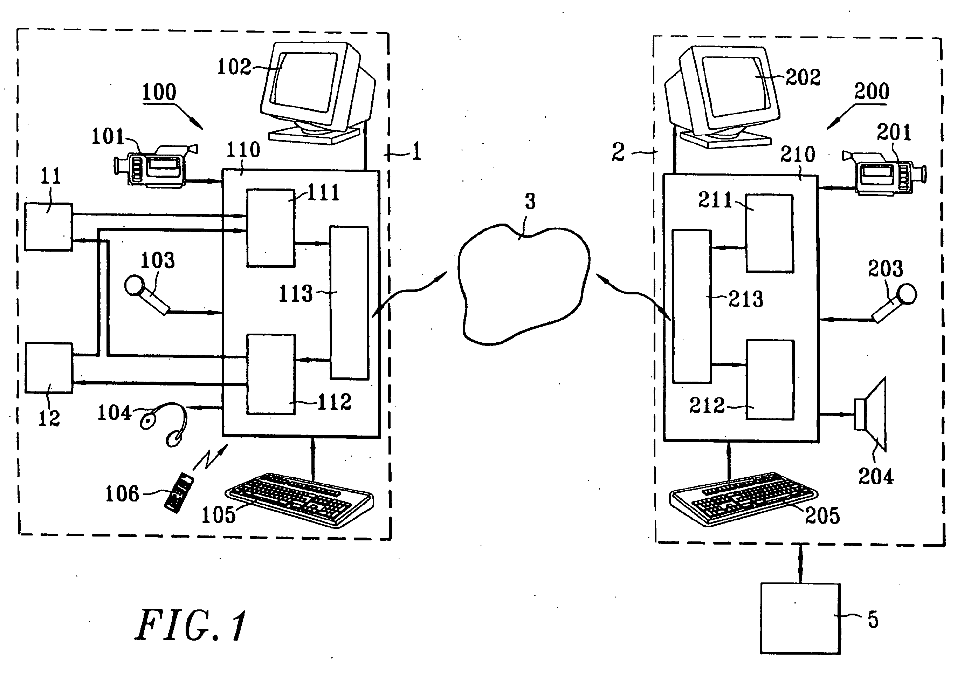

Sur la figure 1 est représenté un système de télé-assistance médicale entre le domicile 1 d'un patient et un centre 2 de soins, un service hospitalier par exemple.

Au domicile 1 du patient sont installés des appareils 11, 12 d'assistance médicale, ainsi qu'un ordinateur-patient 110 équipé de moyens 111 d'acquisition de données en provenance desdits appareils 11, 12. Il s'agit essentiellement d'une mémoire recevant par l'intermédiaire d'un convertisseur analogique/numérique des signaux, en général des tensions, fournis par des capteurs de mesure placés sur les appareils. Inversement, des moyens 112 de transmission d'ordres de commande permettent de modifier les paramètres de fonctionnement des appareils 11, 12. Bien entendu, ces moyens 112 de transmission peuvent utiliser le même convertisseur analogique/numérique que les moyens 111 d'acquisition. Enfin, des moyens 113, tels qu'un modem, assurent la connexion de l'ordinateur-patient 110 à une station-soignant 200 à travers un réseau 3 de télécommunication, par exemple le réseau Numéris 128Kbits/s.

Sur la figure 1, on remarque également la présence au domicile 1 du patient d'une caméra vidéo-patient 101 télécommandable, d'un écran 102 de visualisation pour recevoir des images en provenance de la station-soignant 200, et des moyens de communication phonique constitués d'un microphone 103, éventuellement télécommandable, et d'un moyen de reproduction, de préférence un casque audio 104 sans fil assurant la confidentialité des propos reçus de la station-soignant 200.

De manière optionnelle, l'ordinateur-patient 110 est muni d'un clavier classique filaire, d'un clavier virtuel sur l'écran 102, d'une tablette graphique ou encore un clavier 105 sans fil, ceci pour tenir compte du cas des patients ayant des difficultés d'élocution comme les trachéotomisés.

Enfin, la station-patient 100 est activable par une télécommande 106 pour permettre au patient d'avoir l'initiative d'un appel vers la station-soignant 200, de décrocher quand un soignant appelle, ou d'interrompre la conversation en cours.

Comme le montre la figure 1, la station-soignant 200 comprend un écran 202 de visualisation apte à recevoir des images provenant de la caméra vidéo-patient 101, une caméra vidéo-soignant 201 pour fournir des images, essentiellement celle du soignant, sur l'écran 102 de visualisation du patient, et des moyens de communication phonique, à savoir un microphone 203 et un haut-parleur 204.

Un ordinateur-soignant 210 est muni de moyens de connexion, modem 213 par exemple, au réseau 3 de télécommunication afin d'établir une communication avec la station-patient 100. Cet ordinateur 210 comporte des moyens 211 de télépilotage qui permettent, d'une manière générale, au soignant d'intervenir sur l'ensemble de l'installation située au domicile du patient, et, en particulier, sur les moyens qui lui fournissent des indications sur l'état du patient et le fonctionnement des appareils 11, 12 d'assistance médicale. C'est ainsi que lesdits moyens de télépilotage sont aptes, d'une part, à télécommander à l'aide d'une manette la caméra vidéo-patient 101 en site, en azimut et en zoom, ceci afin de pouvoir examiner visuellement le patient et inspecter les appareils, et, d'autre part, à envoyer à l'ordinateur-patient 110 les ordres de commande qui seront relayés par les moyens 112 de transmission vers les appareils 11, 12.

Des moyens de contrôle, ou de rétrocontrôle après télépilotage, donnent au soignant la possibilité de vérifier la conformité de l'état réel de l'installation du patient avec l'état souhaité. Ce contrôle peut se faire par visualisation sur l'écran 202 des images fournies par la caméra vidéo-patient 101 et /ou par transfert des données transmises depuis les moyens 111 d'acquisition de l'ordinateur-patient 110 à travers le réseau 3 de télécommunication.

Enfin, la station-soignant 200 peut être connectée à des moyens de saisie (scanner, appareil photographique numérique, etc) d'informations pour une utilisation locale ou destinées à être transmises à la station-patient 100. De cette manière, un soignant pourra par exemple communiquer une ordonnance ou un résultat d'analyse à un médecin présent au domicile du patient.

Notons que, par défaut, les stations 100, 200 sont configurées pour communiquer en point à point, c'est-à-dire deux à deux. Dans ce cas, le patient communique toujours avec la même station-soignant. Mais il est également possible que la communication venant du patient soit aiguillée manuellement ou automatiquement vers une des stations-soignant disponibles dans un même service hospitalier référent. Par contre, un même soignant peut communiquer avec chacun des patients dont il a la charge. Toutefois, on peut envisager qu'une station-patient communique aussi avec d'autres stations-soignant que celle désignée par défaut. Par exemple, un patient pourrait communiquer avec un cabinet infirmier, station-soignant par défaut, mais aussi avec son médecin traitant équipé de la même station que le cabinet infirmier.

De plus, selon le choix du réseau 3 de télécommunication, l'utilisation de ponts RNIS ou de réflecteurs IP par exemple permettent la gestion de communications multipoint entre les stations. C'est ainsi qu'une concertation entre un soignant d'un cabinet, le médecin traitant et le patient peut avoir lieu.

La figure 1 montre que la station-soignant 200 est reliée à un équipement téléphonique 5 annexe, pouvant se trouver à quelques mètres, dans le but de permettre à un secrétariat de recevoir des communications phoniques en provenance de la station-patient 100.

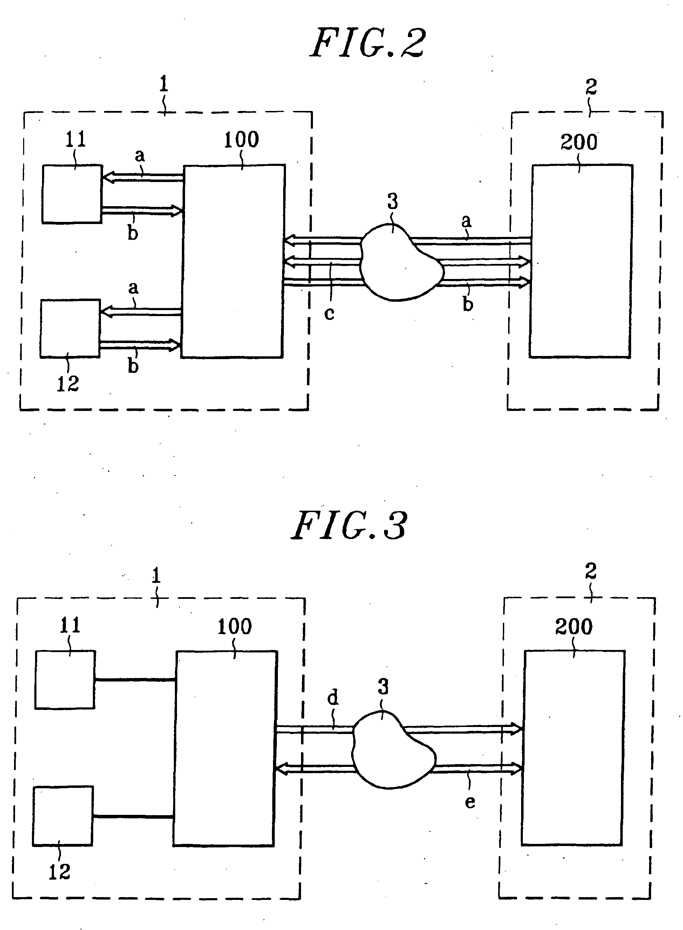

Le fonctionnement général du système de télé-assistance de la figure 1 est illustré sur le schéma de la figure 2.

Selon la pathologie du patient, son médecin lui a prescrit un certain traitement, lequel est administré en l'occurrence par l'utilisation d'appareils 11, 12 de télé-assistance médicale : pompe à nutrition, pompe à morphine, concentrateur d'oxygène par exemple. Ces appareils sont programmés en conséquence et peuvent fonctionner de manière autonome. En plus cette gestion locale, une gestion à distance de chacun des appareils est organisée par la mise en communication d'une station-patient 100 installée chez le patient et d'une station-soignant 200 installée au centre 2 de soins.

Les appareils 11, 12 étant prêts à fonctionner ou en cours de fonctionnement au domicile 1, le soignant peut envoyer à distance (flèches a de la figure 2) des ordres de commande desdits appareils à travers les moyens 211 de télépilotage de l'ordinateur-soignant 210 et les moyens 112 de transmission de l'ordinateur-patient 110. Il peut également (flèches b de la figure 2) contrôler les appareils ou rétrocontrôler les effets des ordres de commande émis, soit en lisant sur son écran 202 les valeurs, mesures, ou accusés de réception, délivrés électroniquement depuis les moyens 111 d'acquisition de la station-patient 100, soit en regardant directement les appareils par télécommande de la caméra vidéo-patient 101. Il est alors possible, selon l'appareil, de lire l'afficheur ou de prendre connaissance de toute indication tirée de l'observation de l'appareil en fonctionnement. En jouant sur la caméra 101, le soignant peut en même temps contrôler le fonctionnement global de l'installation (inspection des tubulures, etc) et/ou discuter par visiophonie (flèche c de la figure 2) avec le patient et aussi voir ses réactions, lui parler pendant les actions effectuées sur les appareils,...

Il faut également signaler qu'en plus des ordres de commande directs des appareils, le soignant a la possibilité, si ces appareils ne peuvent être programmés de façon satisfaisante, de paramétrer un programme sur l'ordinateur-patient 110 qui aura la charge d'exécuter l'administration du traitement médical dans le temps (variations du débit gazeux dans le cadre d'une oxygénothérapie, par exemple). Ce programme peut être paramétré à distance depuis la station-soignant 200 ou sur place à l'occasion d'une visite.

L'initiative de la communication peut aussi provenir de la station-patient 100, soit sur appel volontaire du patient souhaitant entrer en contact avec le soignant pour une raison quelconque, soit lors d'une alerte ou d'une alarme. C'est cette deuxième éventualité qui va maintenant être décrite en regard de la figure 3.

Alors qu'aucune communication visiophonique n'est établie entre le patient et le soignant, la station-patient 100 continue de surveiller de façon autonome le fonctionnement des appareils médicaux 11, 12. Ainsi, si une alarme est déclenchée sur un écart de fonctionnement, une panne ou une alerte d'un 11 des appareils, ou si l'appareil 11 ne peut administrer le traitement comme initialement programmé (manque de produit, obstruction des tubulures, etc) la station-patient 100 établit une communication avec la station-soignant 200 qui, selon les préférences du soignant, peut consister à envoyer (flèche d de la figure 3) un message d'alerte/alarme avec indication de la raison de l'alerte/alarme ou à déclencher (flèche e de la figure 3) un appel visiophonique, supplantant l'appel volontaire du patient, avec affichage côté soignant de la raison de l'appel.

Une fois la communication établie, le soignant prend connaissance de l'environnement dans lequel se trouve le patient et peut en fonction des situations :
- envoyer de nouveaux ordres de commande à l'appareil 11, y compris lorsque cela est possible arrêter les éventuelles sonneries d'alarme persistantes,
- guider quelqu'un présent au domicile 1 du patient de façon à remédier à l'alarme, par exemple enlever un objet tombé obstruant une tubulure, actionner une purge en cas de bulle dans une tubulure,...,
- soutenir le patient ou son entourage en attendant l'arrivée sur place de personnel compétent.

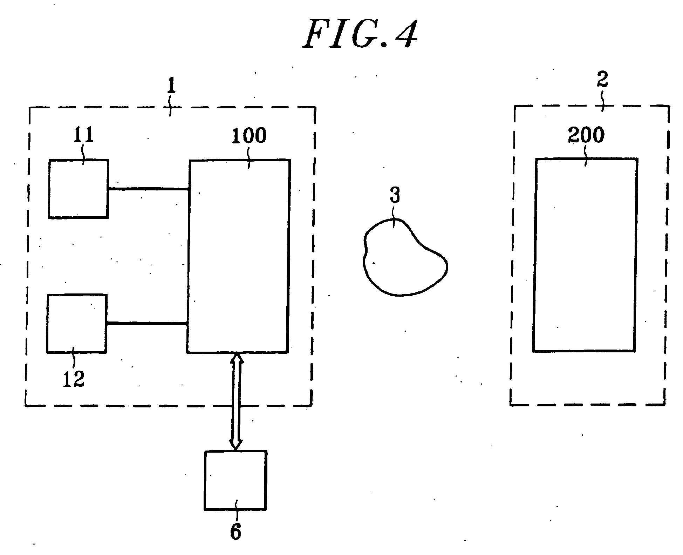

La figure 4 illustre la manière dont le système de l'invention prend en compte un fonctionnement en mode dégradé.

Un mode dégradé survient lorsque la station-patient 100 ne peut entrer en communication avec la station-soignant 200, par exemple hors des horaires et des jours d'ouverture du service d'hospitalisation à domicile. Dans ce cas, afin d'assurer la continuité des soins, la station-patient 100 initie (flèche f de la figure 4) une communication phonique avec un centre-relais 4 qui ne bénéficie pas de l'équipement d'une station mais dispose simplement d'un combiné téléphonique. Ce centre-relais 4 est en général un service d'intervention médical d'urgence.

La raison de l'alerte ou de l'alarme est fournie au soignant du centre-relais 4 par un générateur vocal. Eventuellement, le soignant peut entrer en communication avec le patient en appuyant sur une touche particulière de son combiné téléphonique suite à une indication dudit générateur vocal.

## Revendications

1. Système de télé-assistance médicale à domicile d'un patient, **caractérisé en ce que** ledit système comprend :
- une station-patient (100) comprenant, au moins,
- une caméra vidéo-patient (101) télécommandable, utilisée pour fournir des images d'appareils (11, 12) d'assistance médicale, et
- un ordinateur-patient (110) équipé,
- de moyens (111) d'acquisition de données en provenance desdits appareils (11, 12) d'assistance médicale,
- de moyens (112) de transmission d'ordres de commande auxdits appareils, et,
- de moyens (113) de connexion à un réseau (3) de télécommunication,
- une station-soignant (200) comprenant, au moins,
- un écran (202) de visualisation apte à recevoir des images en provenance de ladite caméra vidéo-patient (101), et
- un ordinateur-soignant (210) muni
- de moyens (213) de connexion audit réseau (3) de télécommunication afin d'établir une communication avec la station-patient (100),
- de moyens (211) de télépilotage de la station-patient aptes à télécommander la caméra vidéo-patient (101) et à envoyer à l'ordinateur-patient (110) lesdits ordres de commande, et
- de moyens (212) de contrôle et de rétrocontrôle après télépilotage des effets desdits ordres de commandes émis, par visualisation sur ledit écran (202) de visualisation des images desdits appareils fournies par la caméra vidéo-patient (101) et par transfert dans l'ordinateur-soignant (210) des données transmises depuis les moyens (111) d'acquisition de l'ordinateur-patient à travers le réseau (3) de télécommunication.

2. Système de télé-assistance selon la revendication 1, **caractérisé en ce que** la station-patient (100) comprend également un écran (102) de visualisation apte à recevoir des images en provenance d'une caméra vidéo-soignant (201) située à la station-soignant (200).

3. Système de télé-assistance selon l'une des revendications 1 ou 2, **caractérisé en ce que** la station-patient (100) et la station-soignant (200) comprennent des moyens (103, 104 ; 203 ; 204) de communication phonique.

4. Système de télé-assistance selon la revendication 3, **caractérisé en ce que** les moyens de communication phonique de la station-patient (100) comprennent un microphone (103) télécommandé par les moyens (211) de télépilotage de la station-soignant (200).

5. Système de télé-assistance selon l'une des revendications 3 ou 4, **caractérisé en ce que** les moyens de communication phonique de la station-patient (100) comprennent un casque audio (104) sans fil.

6. Système de télé-assistance selon l'une quelconque des revendications 1 à 5, **caractérisé en ce que** ledit système comprend en outre un centre-relais (4) apte à entrer en communication phonique avec un générateur vocal au domicile (1) du patient.

7. Système de télé-assistance selon l'une quelconque des revendications 1 à 6, **caractérisé en ce que** la station-soignant (200) est connectée à des moyens de saisie d'informations destinées à une utilisation locale ou à être transmises à la station-patient (100) par l'ordinateur-soignant (210) à travers le réseau (3) de télécommunication.

8. Système de télé-assistance selon l'une quelconque des revendications 3 à 7, **caractérisé en ce que** la station-soignant (200) est reliée à un équipement téléphonique (5) annexe pour recevoir des communications phoniques en provenance de la station-patient (100).

9. Système de télé-assistance selon l'une quelconque des revendications 1 à 8, **caractérisé en ce que** la station-patient (100) est activable au moyen d'une télécommande (106).

10. Système de télé-assistance selon l'une quelconque des revendications 1 à 9, **caractérisé en ce que** l'ordinateur-patient (110) est muni d'un clavier filaire, d'un clavier virtuel sur l'écran (102) de visualisation, d'une tablette graphique, ou d'un clavier (105) sans fil.

11. Système de télé-assistance selon l'une quelconque des revendications 1 à 10, **caractérisé en ce que** le système est adapté à l'établissement d'une communication avec la station-soignant (200) déclenché volontairement par le patient.

12. Système de télé-assistance selon l'une quelconque des revendications 1 à 10, **caractérisé en ce que** le système est adapté à l'établissement d'une communication avec la station-soignant (200) déclenché par une alarme sur un écart de fonctionnement, une panne ou une alerte d'un desdits appareils (11, 12) d'assistance médicale.

13. Station-soignant (200) pour un système de télé-assistance médicale à domicile d'un patient comprenant une station-patient, la station-soignant comprenant,
- un écran (202) de visualisation apte à recevoir, en provenance d'une caméra vidéo-patient (101) télécommandable de la station-patient, des images d'appareils (11, 12) d'assistance médicale, et
- un ordinateur-soignant (210) muni
- de moyens (213) de connexion à un réseau (3) de télécommunication afin d'établir une communication avec la station-patient (100),
- de moyens (211) de télépilotage de la station-patient aptes à télécommander la caméra vidéo-patient (101) et à envoyer à l'ordinateur-patient (110) des ordres de commande destinés à être transmis par un ordinateur-patient de la station-patient auxdits appareils d'assistance médicale, et
- de moyens (212) de contrôle et de rétrocontrôle après télépilotage des effets desdits ordres de commandes, par visualisation sur ledit écran (202) de visualisation des images desdits appareils fournies par la caméra vidéo-patient (101) et par transfert dans l'ordinateur-soignant (210) de données, provenant desdits appareils (11, 12) et transmises depuis des moyens (111) d'acquisition dudit ordinateur-patient à travers le réseau (3) de télécommunication.

## Claims

1. System for home-based medical tele-assistance of a patient, **characterized in that** said system comprises:
- a patient-station (100) comprising, at least,
- a remotely-controllable patient-video camera (101), used to provide images of medical assistance apparatus (11,12) and
- a patient-computer (110) equipped with,
- means (111) for acquiring data originating from said medical assistance apparatus (11,12),
- means (112) for transmitting control commands to said apparatus, and,
- means (113) for connecting to a telecommunication network (3),
- a carer-station (200) comprising, at least,
- a viewing screen (202) able to receive images originating from said patient-video camera (101), and
- a carer-computer (210) furnished with
- means (213) for connecting to said telecommunication network (3) so as to establish a communication with the patient-station (100),
- means (211) for telecommanding the patient-station, able to remotely control the patient-video camera (101) and to dispatch said control commands to the patient-computer (110), and
- means (212) for checking and retrochecking after telecommand the effects of said control commands issued, by viewing on said viewing screen (202) the images of said apparatus provided by the patient-video camera (101) and by transferring to the carer-computer (210) the data transmitted from the acquisition means (111) of the patient-computer through the telecommunication network (3).

2. Tele-assistance system according to Claim 1, **characterized in that** the patient-station (100) also comprises a viewing screen (102) able to receive images originating from a carer-video camera (201) situated at the carer-station (200).

3. Tele-assistance system according to either of Claims 1 or 2, **characterized in that** the patient-station (100) and the carer-station (200) comprise means (103,104; 203; 204) of phonic communication.

4. Tele-assistance system according to Claim 3, **characterized in that** the means of phonic communication of the patient-station (100) comprise a microphone (103) remotely controlled by the means (211) for telecommanding of the carer-station (200).

5. Tele-assistance system according to either of Claims 3 or 4, **characterized in that** the means of phonic communication of the patient-station (100) comprise a wireless audio headset (104).

6. Tele-assistance system according to any one of Claims 1 to 5, **characterized in that** said system furthermore comprises a relay-centre (4) able to enter into phonic communication with a voice generator at the patient's home (1).

7. Tele-assistance system according to any one of Claims 1 to 6, **characterized in that** the carer-station (200) is connected to means for inputting information that is intended for local use or to be transmitted to the patient-station (100) by the carer-computer (210) through the telecommunication network (3).

8. Tele-assistance system according to any one of Claims 3 to 7, **characterized in that** the carer-station (200) is linked to adjoining telephonic equipment (5) so as to receive phonic communications originating from the patient-station (100).

9. Tele-assistance system according to any one of Claims 1 to 8, **characterized in that** the patient-station (100) is activatable by means of a remote control (106).

10. Tele-assistance system according to any one of Claims 1 to 9, **characterized in that** the patient-computer (110) is furnished with a wired keyboard, with a virtual keyboard on the viewing screen (102), with a graphics tablet, or with a wireless keyboard (105).

11. Tele-assistance system according to any one of Claims 1 to 10, **characterized in that** the system is suitable for the establishment of a communication with the carer-station (200) triggered intentionally by the patient.

12. Tele-assistance system according to any one of Claims 1 to 10, **characterized in that** the system is suitable for the establishment of a communication with the carer-station (200) triggered by an alarm upon an operating departure, a fault or an alert of one of said medical assistance apparatus (11,12).

13. Carer-station (200) for a home-based medical tele-assistance system of a patient, said system comprising a patient-station, the carer-station comprising,
- a viewing screen (202) able to receive, originating from a remotely-controllable patient-video camera (101) of the patient-station, images of medical assistance apparatus (11,12), and
- a carer-computer (210) furnished with
- means (213) for connecting to a telecommunication network (3) so as to establish a communication with the patient-station (100),
- means (211) for telecommanding the patient-station, able to remotely control the patient-video camera (101) and to dispatch control commands to the patient-computer (110), said control commands being intended to be transmitted by a patient-computer of the patient-station to said medical assistance apparatus, and
- means (212) for checking and retrochecking after telecommand the effects of said control commands, by viewing on said viewing screen (202) the images of said apparatus provided by the patient-video camera (101) and by transferring to the carer-computer (210) data originating from said apparatus (11,12) and transmitted from acquisition means (111) of said patient-computer through the telecommunication network (3).

## Patentansprüche

1. Telemedizinisches Versorgungssystem eines Patienten im häuslichen Umfeld, **dadurch gekennzeichnet, dass** das System aufweist:
- eine Patienten-Station (100), die mindestens enthält
- eine fernsteuerbare Patienten-Videokamera (101), die verwendet wird, um Bilder von medizinischen Versorgungsgeräten (11, 12) zu liefern, und
- einen Patienten-Computer (110), ausgestattet mit
- Mitteln (111) zur Erfassung von von den medizinischen Versorgungsgeräten (11, 12) kommenden Daten,
- Mitteln (112) zur Übertragung von Steuerbefehlen an die Geräte, und
- Mitteln (113) zur Verbindung mit einem Telekommunikationsnetzwerk (3),
- eine Pfleger-Station (200), die mindestens aufweist
- einen Anzeigebildschirm (202), der von der Patienten-Videokamera (101) kommende Bilder empfangen kann, und
- einen Pfleger-Computer (210), versehen mit
- Mitteln (213) zur Verbindung mit dem Telekommunikationsnetz (3), um eine Verbindung mit der Patienten-Station (100) aufzubauen,
- Mitteln (211) zur Fernsteuerung der Patienten-Station, die fähig sind, die Patienten-Videokamera (101) fernzusteuern und die Steuerbefehle an den Patienten-Computer (110) zu senden, und
- Mitteln (212) zur Kontrolle und zur Retrokontrolle nach Fernsteuerung der Wirkungen der gesendeten Steuerbefehle durch Anzeige auf dem Anzeigebildschirm (202) der Bilder der Geräte, die von der Patienten-Videokamera (101) geliefert werden, und durch Übertragung in den Pfleger-Computer (210) der ausgehend von den Erfassungsmitteln (111) des Patienten-Computers über das Telekommunikationsnetz (3) übertragenen Daten.

2. Televersorgungssystem nach Anspruch 1, **dadurch gekennzeichnet, dass** die Patienten-Station (100) ebenfalls einen Anzeigebildschirm (102) aufweist, der fähig ist, von einer Pfleger-Videokamera (201) stammende Bilder zu empfangen, die sich in der Pfleger-Station (200) befindet.

3. Televersorgungssystem nach einem der Ansprüche 1 oder 2, **dadurch gekennzeichnet, dass** die Patienten-Station (100) und die Pfleger-Station (200) Sprechverbindungsmittel (103, 104; 203; 204) aufweisen.

4. Televersorgungssystem nach Anspruch 3, **dadurch gekennzeichnet, dass** die Sprechverbindungsmittel der Patienten-Station (100) ein Mikrofon (103) aufweisen, das von den Fernsteuerungsmitteln (211) der Pfleger-Station (200) ferngesteuert wird.

5. Televersorgungssystem nach einem der Ansprüche 3 oder 4, **dadurch gekennzeichnet, dass** die Sprechverbindungsmittel der Patienten-Station (100) einen drahtlosen Audio-Kopfhörer (104) aufweisen.

6. Televersorgungssystem nach einem der Ansprüche 1 bis 5, **dadurch gekennzeichnet, dass** das System außerdem eine Vermittlungsstelle (4) aufweist, die fähig ist, mit einem Sprachgenerator in der Wohnung (1) des Patienten in Sprechverbindung zu treten.

7. Televersorgungssystem nach einem der Ansprüche 1 bis 6, **dadurch gekennzeichnet, dass** die Pfleger-Station (200) mit Mitteln zur Eingabe von Informationen verbunden ist, die für eine lokale Nutzung oder zur Übertragung an die Patienten-Station (100) durch den Pfleger-Computer (210) über das Telekommunikationsnetz (3) bestimmt sind.

8. Televersorgungssystem nach einem der Ansprüche 3 bis 7, **dadurch gekennzeichnet, dass** die Pfleger-Station (200) mit einer zugehörigen Telefoneinrichtung (5) verbunden ist, um von der Patienten-Station (100) kommende Sprechverbindungen zu empfangen.

9. Televersorgungssystem nach einem der Ansprüche 1 bis 8, **dadurch gekennzeichnet, dass** die Patienten-Station (100) mittels einer Fernsteuerung (106) aktiviert werden kann.

10. Televersorgungssystem nach einem der Ansprüche 1 bis 9, **dadurch gekennzeichnet, dass** der Patienten-Computer (110) mit einer drahtgebundenen Tastatur, mit einer virtuellen Tastatur auf dem Anzeigebildschirm (102), mit einem Datentablett oder mit einer drahtlosen Tastatur (105) versehen ist.

11. Televersorgungssystem nach einem der Ansprüche 1 bis 10, **dadurch gekennzeichnet, dass** das System für den absichtlichen Aufbau einer Verbindung mit der Pfleger-Station (200) geeignet ist, die durch den Patienten absichtlich ausgelöst wird.

12. Televersorgungssystem nach einem der Ansprüche 1 bis 10, **dadurch gekennzeichnet, dass** das System für den Aufbau einer Verbindung mit der Pflege-Station (200) geeignet ist, die durch einen Alarm bei einer Betriebsverzögerung, eine Panne oder eine Alarmanzeige der medizinischen Versorgungsgeräte (11, 12) ausgelöst wird.

13. Pflege-Station (200) für ein telemedizinisches Versorgungssystem eines Patienten im häuslichen Umfeld, wobei das Versorgungssystem eine Patienten-Station aufweist, wobei die Pfleger-Station aufweist
- einen Anzeigebildschirm (202), der von einer fernsteuerbaren Patienten-Videokamera (101) der Patienten-Station kommende Bilder von medizinischen Versorgungsgeräten (11, 12) empfangen kann, und
- einen Pfleger-Computer (210), versehen mit
- Mitteln (213) zur Verbindung mit einem Telekommunikationsnetz (3), um eine Verbindung mit der Patienten-Station (100) aufzubauen,
- Mitteln (211) zur Fernsteuerung der Patienten-Station, die fähig sind, die Patienten-Videokamera (101) fernzusteuern und an den Patienten-Computer (110) Steuerbefehle zu senden, die dazu bestimmt sind, von einem Patienten-Computer der Patienten-Station an die medizinischen Versorgungsgeräte übertragen zu werden, und
- Mitteln (212) zur Kontrolle und zur Retrokontrolle nach Fernsteuerung der Wirkungen der Steuerbefehle durch Anzeige auf dem Anzeigebildschirm (202) der Bilder der Geräte, die von der Patienten-Videokamera (101) geliefert werden, und durch Übertragung in den Pfleger-Computer (210) von von den Geräten (11, 12) kommenden und von Erfassungsmitteln (111) des Patienten-Computers über das Telekommunikationsnetz (3) übertragenen Daten.
